# EUROPEAN PATENT SPECIFICATION

(11) **EP 1 835 035 B1**
(45) Date of publication and mention of the grant of the patent: **17.02.2010**
(21) Application number: 06005241.2
(22) Date of filing: 15.03.2006
(51) Int. Cl.: C12P 19/40, C12N 11/08, C12N 15/70

(54) **A process for immobilizing cells on a resin**
Verfahren zur Immobilisierung von Zellen auf Harzen
Procédé pour l'immobilisation de cellules sur une résine

(43) Date of publication of application: 19.09.2007
(73) Proprietor: EXPLORA Laboratories SA, 6850 Mendrisio (CH)
(72) Inventor: Zuffi, Gabriele, 21100 Varese (IT); Monciardini, Simone, 21100 Varese (IT)
(74) Representative: Zardi, Marco

(56) References cited:
- EP-A- 0 361 165
- EP-A- 1 439 220
- WO-A-00/39307
- WO-A-97/44478
- SPOLDI E ET AL: "RECOMBINANT BACTERIAL CELLS AS EFFICIENT BIOCATALYSTS FOR THE PRODUCTION OF NUCLEOSIDES" NUCLEOSIDES, NUCLEOTIDES AND NUCLEIC ACIDS, TAYLOR & FRANCIS, PHILADELPHIA, PA, US, vol. 20, no. 4-7, April 2001 (2001-04), pages 977-979, XP009009766 ISSN: 1525-7770

### SEQUENCE LISTING

<110> EXPLORA LABORATORIES SA
<120> A PROCESS FOR IMMOBILIZING CELLS ON RESINS
<130> EXP001BEP
<160> 2
<170> PATENTIN VERSION 3.3
<210>
   <211> 4454
   <212> DNA
   <213> ARTIFICIAL
<220>
   <223> PLASMID
<220>
   <221> GENE
   <222> (49)..(843)
   <223> KANAMICINE RESISTANCE
<220>
   <221> GENE
   <222> (1285)..(2046)
   <223> UDP
<400> 1
<210> 2
   <211> 4393
   <212> DNA
   <213> ARTIFICIAL
<220>
   <223> PLASMID
<220>
   <221> GENE
   <222> (438)..(1232)
   <223> KANAMICINE RESISTANCE
<220>
   <221> GENE
   <222> (1277)..(1977)
   <223> DEOD
<400> 2
   TGTAAAACGA CGGCCAGTGC CAAGCTTGCA TGCCTGCAGG TCGACTCTAG AGGATCCCCG 60
   GGTACCGAGC TCGAATTCCG CTAGCTTCAC GCTGCCGCAA GCACTCAGGG CGCAAGGGCT 120
   GCTAAAGGAA GCGGAACACG TAGAAAGCCA GTCCGCAGAA ACGGTGCTGA CCCCGGATGA 180
   ATGTCAGCTA CTGGGCTATC TGGACAAGGG AAAACGCAAG CGCAAAGAGA AAGCAGGTAG 240
   CTTGCAGTGG GCTTACATGG CGATAGCTAG ACTGGGCGGT TTTATGGACA GCAAGCGAAC 300
   CGGAATTGCC AGCTGGGGCG CCCTCTGGTA AGGTTGGGAA GCCCTGCAAA GTAAACTGGA 360
   TGGCTTTCTT GCCGCCAAGG ATCTGATGGC GCAGGGGATC AAGATCTGAT CAAGAGACAG 420
   GATGAGGATC GTTTCGCATG ATTGAACAAG ATGGATTGCA CGCAGGTTCT CCGGCCGCTT 480
   GGGTGGAGAG GCTATTCGGC TATGACTGGG CACAACAGAC AATCGGCTGC TCTGATGCCG 540
   CCGTGTTCCG GCTGTCAGCG CAGGGGCGCC CGGTTCTTTT TGTCAAGACC GACCTGTCCG 600
   GTGCCCTGAA TGAACTCCAA GACGAGGCAG CGCGGCTATC GTGGCTGGCC ACGACGGGCG 660
   TTCCTTGCGC AGCTGTGCTC GACGTTGTCA CTGAAGCGGG AAGGGACTGG CTGCTATTGG 720
   GCGAAGTGCC GGGGCAGGAT CTCCTGTCAT CTCACCTTGC TCCTGCCGAG AAAGTATCCA 780
   TCATGGCTGA TGCAATGCGG CGGCTGCATA CGCTTGATCC GGCTACCTGC CCATTCGACC 840
   ACCAAGCGAA ACATCGCATC GAGCGAGCAC GTACTCGGAT GGAAGCCGGT CTTGTCGATC 900
   AGGATGATCT GGACGAAGAG CATCAGGGGC TCGCGCCAGC CGAACTGTTC GCCAGGCTCA 960
   AGGCGCGGAT GCCCGACGGC GAGGATCTCG TCGTGACCCA TGGCGATGCC TGCTTGCCGA 1020
   ATATCATGGT GGAAAATGGC CGCTTTTCTG GATTCATCGA CTGTGGCCGG CTGGGTGTGG 1080
   CGGACCGCTA TCAGGACATA GCGTTGGCTA CCCGTGATAT TGCTGAAGAG CTTGGCGGCG 1140
   AATGGGCTGA CCGCTTCCTC GTGCTTTACG GTATCGCCGC TCCCGATTCG CAGCGCATCG 1200
   CCTTCTATCG CCTTCTTGAC GAGTTCTTCT GAGCGGGACT CTGGGGTTCG CCCAAGCTTG 1260
   CATGCCTGCA GGTCGACTTA CTCTTTATCG CCCAGCAGAA CGGATTCCAG TGCGATTTTG 1320
   ATCATGTCGT TGAAGGTAGT CTGACGCTCA GCGGCAGTGG TCTGCTCGTG AGTGCGGATG 1380
   TGGTCAGATA CGGTGCAGAT GGTCAGGGCT TTCGCGCCAA ATTCTGCAGC GACGCCGTAG 1440
   ATACCAGCCG CTTCCATTTC CACGCCGAGA ATGCCGTATT TTTCCATCAC GTCGAACATT 1500
   TCGCCGTCCG GAGAGTAGAA CAGGTCAGCG GAGAACAGGT TACCCACGCG AGCATCAATA 1560
   CCCAGTGCTT TAGCTGCATC TACTGCGTTA CGCACCATGT CGAAGTCAGC GATAGCGGCA 1620
   AAGTCATGGT CTTTAAAACG GATGCGGTTA ACTTTGGAAT CGGTGCAGGC ACCCATACCG 1680
   ATAACGACGT CGCGCAGTTT TACGTGCGGC AGAACTGCGC CACAGGAACC CACGCGGATA 1740
   ATTTTCTTCA CGCCGAAATC GGTGATCAGT TCTTTGGTGT AGATGGAGCA GGACGGGATA 1800
   CCCATACCGT GACCCATTAC GGAAATTTTG CGGCCTTTGT AAGTACCGGT GAAGCCCAGC 1860
   ATACCGCGAA CGTTGTTCAC TTCACGGGCA TCTTCAAGGA AAGTTTCAGC AATATACTTC 1920
   GCACGCAGCG GGTCGCCTGG CATCAAAACT ACGTCAGCGA AATCGCCCAT TTCTGCATTA 1980
   ATGTGTGGGG TAGCCATGGA AGAATTCGTA ATCATGGTCA TAGCTGTTTC CTGTGTGAAA 2040
   TTGTTATCCG CTCACAATTC CACACAACAT ACGAGCCGGA AGCATAAAGT GTAAAGCCTG 2100
   GGGTGCCTAA TGAGTGAGCT AACTCACATT AATTGCGTTG CGCTCACTGC CCGCTTTCCA 2160
   GTCGGGAAAC CTGTCGTGCC AGCTGCATTA ATGAATCGGC CAACGCGCGG GGAGAGGCGG 2220
   TTTGCGTATT GGGCGCTCTT CCGCTTCCTC GCTCACTGAC TCGCTGCGCT CGGTCGTTCG 2280
   GCTGCGGCGA GCGGTATCAG CTCACTCAAA GGCGGTAATA CGGTTATCCA CAGAATCAGG 2340
   GGATAACGCA GGAAAGAACA TGTGAGCAAA AGGCCAGCAA AAGGCCAGGA ACCGTAAAAA 2400
   GGCCGCGTTG CTGGCGTTTT TCCATAGGCT CCGCCCCCCT GACGAGCATC ACAAAAATCG 2460
   ACGCTCAAGT CAGAGGTGGC GAAACCCGAC AGGACTATAA AGATACCAGG CGTTTCCCCC 2520
   TGGAAGCTCC CTCGTGCGCT CTCCTGTTCC GACCCTGCCG CTTACCGGAT ACCTGTCCGC 2580
   CTTTCTCCCT TCGGGAAGCG TGGCGCTTTC TCATAGCTCA CGCTGTAGGT ATCTCAGTTC 2640
   GGTGTAGGTC GTTCGCTCCA AGCTGGGCTG TGTGCACGAA CCCCCCGTTC AGCCCGACCG 2700
   CTGCGCCTTA TCCGGTAACT ATCGTCTTGA GTCCAACCCG GTAAGACACG ACTTATCGCC 2760
   ACTGGCAGCA GCCACTGGTA ACAGGATTAG CAGAGCGAGG TATGTAGGCG GTGCTACAGA 2820
   GTTCTTGAAG TGGTGGCCTA ACTACGGCTA CACTAGAAGA ACAGTATTTG GTATCTGCGC 2880
   TCTGCTGAAG CCAGTTACCT TCGGAAAAAG AGTTGGTAGC TCTTGATCCG GCAAACAAAC 2940
   CACCGCTGGT AGCGGTGGTT TTTTTGTTTG CAAGCAGCAG ATTACGCGCA GAAAAAAAGG 3000
   ATCTCAAGAA GATCCTTTGA TCTTTTCTAC GGGGTCTGAC GCTCAGTGGA ACGAAAACTC 3060
   ACGTTAAGGG ATTTTGGTCA TGAGATTATC AAAAAGGATC TTCACCTAGA TCCTTTTAAA 3120
   TTAAAAATGA AGTTTTAAAT CAATCTAAAG TATATATGAG TAAACTTGGT CTGACAGTTA 3180
   CCAATGCTTA ATCAGTGAGG CACCTATCTC AGCGATCTGT CTATTTCGTT CATCCATAGT 3240
   TGCCTGACTC CCCGTCGTGT AGATAACTAC GATACGGGAG GGCTTACCAT CTGGCCCCAG 3300
   TGCTGCAATG ATACCGCGAG ACCCACGCTC ACCGGCTCCA GATTTATCAG CAATAAACCA 3360
   GCCAGCCGGA AGGGCCGAGC GCAGAAGTGG TCCTCCGATC GTTGTCAGAA GTAAGTTGGC 3420
   CGCAGTGTTA TCACTCATGG TTATGGCAGC ACTGCATAAT TCTCTTACTG TCATGCCATC 3480
   CGTAAGATGC TTTTCTGTGA CTGGTGAGTA CTCAACCAAG TCATTCTGAG AATAGTGTAT 3540
   GCGGCGACCG AGTTGCTCTT GCCCGGCGTC AATACGGGAT AATACCGCGC CACATAGCAG 3600
   AACTTTAAAA GTGCTCATCA TTGGAAAACG TTCTTCGGGG CGAAAACTCT CAAGGATCTT 3660
   ACCGCTGTTG AGATCCAGTT CGATGTAACC CACTCGTGCA CCCAACTGAT CTTCAGCATC 3720
   TTTTACTTTC ACCAGCGTTT CTGGGTGAGC AAAAACAGGA AGGCAAAATG CCGCAAAAAA 3780
   GGGAATAAGG GCGACACGGA AATGTTGAAT ACTCATACTC TTCCTTTTTC AATATTATTG 3840
   AAGCATTTAT CAGGGTTATT GTCTCATGAG CGGATACATA TTTGAATGTA TTTAGAAAAA 3900
   TAAACAAATA GGGGTTCCGC GCACATTTCC CCGAAAAGTG CCACCTGACG TCTAAGAAAC 3960
   CATTATTATC ATGACATTAA CCTATAAAAA TAGGCGTATC ACGAGGCCCT TTCGTCTCGC 4020
   GCGTTTCGGT GATGACGGTG AAAACCTCTG ACACATGCAG CTCCCGGAGA CGGTCACAGC 4080
   TTGTCTGTAA GCGGATGCCG GGAGCAGACA AGCCCGTCAG GGCGCGTCAG CGGGTGTTGG 4140
   CGGGTGTCGG GGCTGGCTTA ACTATGCGGC ATCAGAGCAG ATTGTACTGA GAGTGCACCA 4200
   TATGCGGTGT GAAATACCGC ACAGATGCGT AAGGAGAAAA TACCGCATCA GGCGCCATTC 4260
   GCCATTCAGG CTGCGCAACT GTTGGGAAGG GCGATCGGTG CGGGCCTCTT CGCTATTACG 4320
   CCAGCTGGCG AAAGGGGGAT GTGCTGCAAG GCGATTAAGT TGGGTAACGC CAGGGTTTTC 4380
   CCAGTCACGA CGT 4393

## Description

### Field of application

The present invention generally relates to a process for immobilizing cells and to the use of a resin for immobilizing cells.

### Prior Art

Since various decades¹ cell immobilization is a well known technique, used with the aim to improve fermentative and biosynthetic processes. The most commonly used techniques for immobilizing cells are those involving the establishment of covalent bonds between cells and an activated inorganic carrier, as for example silanized² or glutaraldehyde and isocyanate³ treated silica, or a cross-linking between cells by bi- or polifunctional reagents, such as e.g. glutaraldehyde⁴ and toluene diisocyanate, or the trapping in polymer matrixes, as for example agar, alginates, carragenanes, cellulose and its derivatives, collagen, gelatin and polyacrylamide.

A technique which is less used than the previous ones is represented by the adsorption on carriers such as kieselguhr, glass, ceramics and plastic materials, caused by electrostatic interactions (Van der Waals forces) between the carrier and the cells.

An example^{5,6} of cell adsorption on a ionic exchange resin, and precisely the adsorption of *Escherichia coli* cells on a strong anionic exchange resin (Dowex 1), was reported in 1960-61, but afterwards only examples of cell immobilization performed by trapping, generally on carrageen, alginates and polyacrylamides are cited in the literature.

This is probably due to the scarce stability of the immobilizations so far obtained by the adsorption techniques and particularly of the immobilizations on resins.

It would be instead desiderable to make available a process to permanently immobilize cells on resins by adsorption, because in such a way the cells do not undergo any mechanical stress nor any chemical degradation, keep themselves vital and retain at a maximum level their own ability to maintain the enzymes which they produce in optimal physiological conditions and to consequently catalyze reactions that are useful for different biosynthetic or fermentative pathways.

### Summary of the Invention

The problem underlying the present invention was that of providing a process to permanently immobilize cells by adsorption on resins, in such a way that they can be used particularly to perform reactions catalyzed by the enzymes produced by the cells themselves.

Such a problem was solved, according to the invention, by a process for immobilizing cells, which includes an adsorption phase of such cells on a resin wherein said cells are Escherichia coli cells and said resin is the weak anionic exchange resin Duolite A568® (Rohm & Haas) resin.

In the process for immobilizing cells according to the present invention a ratio between wet weight of cells (cell paste) and dry weight of resin comprised between 0.5:1 and 2:1, advantageously about 1:1, is preferably used. The cell paste has a water content comprised between 70 and 80% w/w.

The immobilization is performed operating in a buffered aqueous medium at pH 7.4, preferably using a phosphate buffer with molarity ranging between 0.1 M and 1 M, advantageously equal to about 0.5 M.

The contact time between resin and cells necessary to obtain immobilization of the latter is higher than or equal to 24 hours and preferably higher than or equal to 72 hours.

By the process according to the present invention it is also possible to immobilize at the same time on the resin at least two types of different cells, which are able to produce different enzymes.

In particular, it is possible to co-immobilize Escherichia coli cells producing uridine phosphorylase (UPase) and Escherichia coli cells producing purine nucleoside phosphorylase (PNPase).

In a further aspect, the present invention relates to a process for the production of nucleosides, comprising the reversible reaction of a pentose-1-phosphate with a purine or pyrimidine base, in the presence of cells producing UPase and/or PNPase, immobilized by means of the process described above, wherein said cells are cells of the Escherichia coli species.

The term "nucleosides" is referred to both natural and modified nucleosides, as well as the terms "purine base" and "pyrimidine base" are referred to both natural bases and modified bases.

According to a further embodiment, said cells are co-immobilized on the resin.

In a further aspect, the present invention relates to a process for nucleosides transglycosylation, comprising the reaction between a pyrimidine nucleoside and a purine base or between a purine nucleoside and a pyrimidine base or between a pyrimidine nucleoside and a pyrimidine base which is different from that present in said pyrimidine nucleoside or finally between a purine nucleoside and a purine base which is different from that present in said purine nucleoside, wherein such a reaction occurs in the presence of cells producing UPase and of cells producing PNPase or in the presence of cells producing UPase and PNPase, wherein said cells producing UPase and said cells producing PNPase are Escherichia coli cells and wherein said cells are immobilized by the immobilization process described above.

The use of Escherichia coli cells from the DH5alpha strain transformed by the plasmid vectors having the sequences reported in the Seq. Id. No. 1 and 2. is particularly preferred.

In a further aspect, the present invention relates to a transglycosylation process between 2'-fluoroadenine and arabinofuranosyl uracil to give Fludarabine, wherein such a reaction occurs in the presence of cells producing UPase and of cells producing PNPase or in the presence of cells producing UPase and PNPase, wherein said cells producing UPase and said cells producing PNPase are Escherichia coli cells and wherein said cells are immobilized by the immobilization process described above..

The utilization of Escherichia coli cells of DH5alpha strain transformed by the plasmid vectors having the sequences reported in the Seq. Id. No. 1 and 2 is particularly preferred. Suitably the Escherichia coli cells transformed with the plasmid vectors of the Seq. Id. No. 1 and with the plasmid vectors of the Seq. Id. No. 2 are co-immobilized on the resin.

### Detailed description of the invention

The process for immobilizing cells according to the present invention was achieved during studies and experiments aimed at improving a system of bio-catalysts usable for the production, even on industrial scale, of nucleosides and modified analogues, particularly referring to Fludarabine.

For this purpose, it was decided to use bioconversion reactions, using prokaryotic enzymes, which are preferable because they have a wider substrate specificity, are easily obtainable in large amounts from cellular sources and are well known regarding to their metabolic pathways.

The enzymatic systems that may be used for the biosynthesis of natural and/or modified nucleosides are mainly two: deoxyribosyl transferases (DRTases) and nucleoside phosphorylases (NP)^{7,8}.

Deoxyribosyl transferases have the disadvantage to be very specific for 2-deoxyribose,and for this reason they can only be used for deoxynucleosides in position 2'.

Nucleoside phosphorilases are enzymes, which are present in most bacteria, in yeasts and also in the superior eukaryotes and are divided in two classes based on their quaternary structure, their folding and the kind of substrate they act on.

In particular, the enzymes Uridine Phosphorylase (E.C. 2.4.2.3) and Purine Nucleoside Phosphorylase (E.C. 2.4.2.1) are particularly indicated to perform bioconversion reactions for the production of molecules of industrial interest, by virtue of their high thermal-stability and the possibility they have to work also at high concentrations of solvent.

Unlike DRTases, prokaryotic phosphorylases have a lower substrate specificity regarding the sugar and are able to recognize for example ribose, deoxyribose, dideoxyribose, arabinose and various modifications at the position 2', 3' or 5'. Also with regard to the nitrogen bases, the phosphorylase enzymes have low substrate specificity and are also able to recognize, apart from more or less modified natural bases, other heterocyclic compounds. However, a fundamental requirement is the presence of a nitrogen atom at position 1 of pyrimidines and at position 9 of purines, which binds to the carbon of the sugar once the phosphate has left^{9,10}.

The two enzymes are well known in the literature, both in their aminoacid sequence, which is conserved in different organisms, and from the genetic-molecular point of view. In fact, the genes which encode them are known (UdP for Uridine Phosphorylase and deoD for Purine Nucleoside Phosphorylase) and the associated sequences are available in the appropriate databases.

Both the enzymes belong to the salvage pathways of nucleosides and catalyze, in the presence of phosphate ions, the reversible phosphorolysis of the nucleoside to give sugar-1-phosphate and the corresponding heterocyclic base as reported in the following scheme

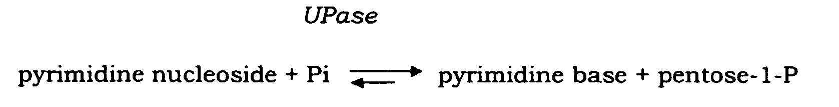

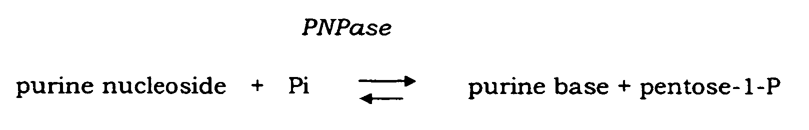

It is possible to transfer the sugar of a nucleoside from a pyrimidine base to another one, using Uridine Phosphorylase, and from a purine base to another one using the Purine Nucleoside Phosphorylase.

Otherwise, using both phosphorylases, it is possible to transfer the sugar from a pyrimidine base to a purine base or vice versa. The equilibrium constants of the two phosphorolysis reactions generally promote the transfer of the sugar from a pyrimidine base to a purine base with high conversion yields.

A scheme of the two coupled reactions is reported:

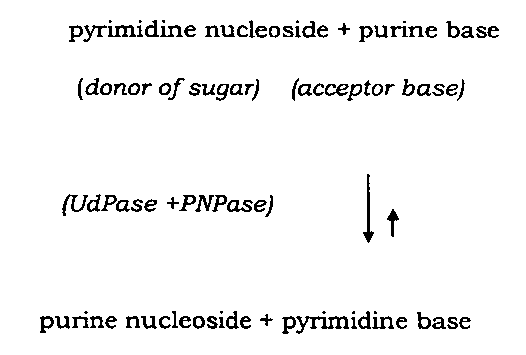

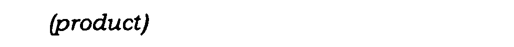

Thus it is made available an efficient method for the production of purine nucleosides, which are generally of greater value and more difficult approach from a chemical point of view^{11,12,13,14}.

This synthetic pathway shows different advantages compared with the chemical one, including stereospecificity and stereoselectivity (with the ensuing higher yield), the use of more gentle reaction conditions, in an aqueous environment with lower environmental impact. Moreover, it does not require long and expensive reactions for protecting and deprotecting labile groups.

On the other hand, the disadvantages are the relatively low solubility of the substrates and the low specific activity of the procaryotic cells. To obviate the latter disadvantage, recombinant strains able to overproduce the phosphorylase enzymatic activities have already been constructed and described in the literature, also with the two enzymes over-expressed in the same cell^{15,16}.

Transglycosylation reactions (transfer of the sugar from one base to another) by means of bio-catalysts have already been described. In these reactions, whole cells or more or less purified and immobilized enzymes were used^{17,18,19}.

When non-supported whole cells are used, it is not possible to re-use them unless they are retained by means of micro- or ultrafiltration membranes, but such a method requires dedicated plants and very large operating volumes, involves possible precipitation problems with scarcely soluble products and can easily cause a cell lysis because of the operating pressures. Also the enzyme immobilization needs long and expensive processes involving cell breakage, recovery of the enzymes and removal of cellular debris, operations that are not easily carried out on a large scale and that require expensive and dedicated plants.

The method for immobilizing bacterial cells described in the present invention allows obtaining a biocatalyst with a high specific activity, which can be re-used for numerous cycles and is able to work at high temperatures also in the presence of high concentration of organic solvent.

The extreme simplicity of the method described in the present invention makes the process to obtain a biocatalyst with such characteristics much more cost-effective, as it does not need dedicated plants but only tools of general use, and notably reduces the preparation times and the number of necessary steps.

The possibility to perform nucleoside biosynthesis reactions by means of phosphorylase producing cells, immobilized on a resin, was initially verified on wild type Escherichia coli strains.

First of all, the ability of such strains to catalyze the nucleoside biosynthesis was verified as follows.

### Reaction of Fludarabine with wild type Escherichia coli

To 2940 ml of 5 mM 2'-Fluoro-adenine solution; 7.5 mM ara-U (arabinofuranosyl-uracil); 30 mM KH₂PO₄ at pH 7.0 in 4% DMSO and exactly termostated at 60°C, 60 ml of 47.5% cell suspension (wet weight/volume) of wild type Escherichia coli are added.

Following 6 days incubation, the reaction mixture is filtered, thus removing the cells. The Fludarabine base is precipitated from water after concentration of the filtered material. The bioconversion yield is about 68%.

The Escherichia coli cells were then immobilized as follows.

### 1. Immobilization of wild type Escherichia coli

Initially we proposed to evaluate the possibility to immobilize enzymes extracted from Escherichia coli cells by means of a resin.

In order to do this, a suspension of cell paste of 10% Escherichia coli (wet weight/volume) in 1 M potassium phosphate buffer at pH 7.5 is divided in three aliquots. The first aliquot (10 ml) is used as a control while the other two aliquots (10 ml each) undergo respectively 3 and 6 subsequent cycles alternated between freeze at -20°C and defrost at 50°C to cause the breakage of the cell-wall. 1 gram of Duolite A568 (Rohm & Haas) dry resin is added to each aliquot of cell suspension, and is left in contact under mild rotational stirring 4 days at room temperature.

After the above incubation, the resin is filtered and washed thoroughly with deionized water until a perfectly clear filtrate is obtained.

About 2.5 grams of wet resin are obtained from each test. The activity of the immobilized resin is controlled by a standard reaction requiring the coupling of the two phosphorylase activities. The reaction, starting from Ara-U and Adenine, in the presence of phosphate ions, causes the formation of the new nucleoside Ara-A (arabinose-adenine), obtained by addition of adenine to the arabinose sugar, and of the Uracil base released by phosphorolysis of Ara-U.

1 ml of the following reaction mixture termostated at 60°C is added to 100 or 200 mg aliquots of wet resin: 5 mM Ara-U; 5 mM Adenine: 30 mM KH₂PO₄ at pH 7.1.

Incubation under stirring is performed and the three reactions are controlled after the same incubation time.

| **(2 h) Sample** | **Adenine →Ara-A conversion** | **Ara-U → Uracil conversion** |
|---|---|---|
| 100 mg Control | 11.4% | 16.2% |
| 200 mg Control | 24.0% | 37.6% |
| 100 mg 3 freezing/defrost cycles | 6.8% | 7.2% |
| 200 mg 3 freezing/defrost cycles | 10.2% | 10.6% |
| 100 mg 6 freezing/defrost cycles | 4.0% | 5.2% |
| 200 mg 6 freezing/ defrost cycles | 6.9% | 8.7% |

The reactions are controlled after 1 day.

| **Sample 24 h** | **Adenine →Ara-A conversion** | **Ara-U → Uracil conversion** |
|---|---|---|
| 200 mg Control | 68.20% | 81.5% |
| 200 mg 3 freezing/defrost cycles | 64.2% | 51.0% |
| 200 mg 6 freezing/defrost cycles | 58.0% | 42.0% |

The untreated cell suspension unexpectedly showed a definitely higher activity compared with those submitted to freezing and defrost cycles.

The same resin samples were submitted to subsequent reactions to see if they succeeded in maintaining the enzymatic activities also in subsequent cycles.

| **Sample 2° reaction 2h** | **Adenine →Ara-A conversion** | **Ara-U → Uracil conversion** |
|---|---|---|
| 200 mg Control | 18.2% | 14.2% |
| 200 mg 3 freezing/ defrost cycles | 12.8% | 8.1% |
| 200 mg 6 freezing/defrost cycles | 9.8% | 6.3% |

| **Sample 3° reaction 2h** | **Adenine →Ara-A conversion** | **Ara-U → Uracil conversion** |
|---|---|---|
| 200 mg Control | 27.0% | 19.4% |
| 200 mg 3 freezing/defrost cycles | 14.4% | 10.1% |
| 200 mg 6 freezing/defrost cycles | 11.5% | 8.0% |

The activities were maintained in all the tests and all the reactions; under the same working conditions, the control had at least about a double activity.

Afterwards, the compatibility of such immobilized cells with high concentrations of organic solvents in the reaction mixtures was verified.

### 2. Reactions to Fludarabine with immobilized resin in different solvents

The resin having the wild type Escherichia coli cells described above immobilized thereon was used for reactions to yield Fludarabine in the presence of DMSO, DMF and THF.

THF is immediately eliminated as 2'-fluoroadenine is pactically insoluble in it, even in very low concentrations.

1 ml of reaction mixture at 60°C consisting of:
4 mM 2-Fluoroadenine; 6 mM Ara-U; 30 mM KH₂PO₄; 20% DMSO or DMF
   is added to 250 mg of resin carrying immobilized E. coli cells

Different reactions are carried out using the same resin.

| **Sample (1^{st} reaction 20 h)** | **2-F-Adenine →Fludarabine** | **Ara-U → Uracil** |
|---|---|---|
| Reaction in DMSO | 70% | 77% |
| Reaction in DMF | 65% | 75% |

| **Sample (2^{nd} reaction 48 h)** | **2-F-Adenine →Fludarabine** | **Ara-U → Uracil** |
|---|---|---|
| Reaction in DMSO | 73.4% | 72.3% |
| Reaction in DMF | 63.6% | 70.5% |

| **Sample (3^{rd} reaction 6 days)** | **2-F-Adenine →Fludarabine** | **Ara-U → Uracil** |
|---|---|---|
| Reaction in DMSO | 72% | 73.7% |
| Reaction in DMF | 68.3% | 76% |

| **Sample (4^{th} reaction 20 h)** | **2-F-Adenine →Fludarabine** | **Ara-U → Uracil** |
|---|---|---|
| Reaction in DMSO | 64% | 59% |
| Reaction in DMF | 68% | 72% |

The resin maintains a fair activity also after 10 total days of permanence in the reaction at 60°C with high concentrations of both solvents.

The new biocatalyst is then easily reusable for different reaction cycles with a very slight activity loss. However, the enzymatic specific activity of the cells to be immobilized was not yet too high and did not allow optimizing the reaction, since still low substrate concentrations had to be used, with a consequent increase in the reaction volumes.

New recombinant strains able to express considerable amounts of UPase or PNPase were then used.

### 3. Construction of Recombinant Strains expressing the UPase enzyme or the PNPase enzyme

The recombinant strains were constructed transforming an Escherichia coli host strain with a high copy number plasmid containing the gene of interest and a marker for the selection.

The host strain used is the DH5alpha strain, which is easily available (GIBCO-BRL) on the market and well described in the literature. It is a strain derived from Escherichia coli K12, which is considered of security class 1 and, therefore, appropriate for an industrial utilization.

The UdP gene, coding for the UPase enzyme, and the deoD gene, coding for the PNPase enzyme, are already well described in the literature and their sequences are known and available in the EMBL databases characterized by the access numbers X15679 for UdP and M60917 for deoD.

Genes were amplified by means of PCR (polymerase chain reaction), using expressly prepared synthetic primers.

Using the appropriate restriction enzymes, KpnI and SalI for UdP and EcoRI and SalI for deoD, the genes were inserted in the poly-linker region of the high copy number plasmid pUC18, which is well characterized in the literature and commercially available.

In both plasmids (the one containing the UdP gene and the one containing the deoD gene) resistance to the antibiotic Kanamycin, obtained by digestion with the pBSL14 plasmid restriction enzyme HindIII, commercially available, was then inserted.

Finally, in both plasmids (the one containing the UdP gene and the one containing the deoD gene) Ampicillin resistance was eliminated by deletion, through digestion with the enzyme AvaII.

Unexpectedly, two sites recognized by the restriction enzyme AvaII were found with the subsequent formation of 3 plasmid fragments instead of the expected two, whereas in the literature only one restriction site for this enzyme is reported.

The final plasmids were obtained by recovering the two longer fragments and removing the unnecessary fragment that had formed. In the following table the main characteristics of the new genetically modified strains are reported

**TABLE**

| STRAIN | Host | Plasmid | Marker selection | Expressed protein | AmpR presence |
|---|---|---|---|---|---|
| EXP05/03 | DH5alpha | pUC 18 | Kanamycin | UPase | No |
| EXP05/04 | DH5alpha | pUC18 | Kanamycin | PNPase | No |

The sequences of the plasmids in the above table are reported in the lists at the end of the description and particularly the sequence of the pUC 18 plasmid containing the UdP gene corresponds to the Seq. Id. No. 1 and the sequence of the pUC 18 plasmid containing the deoD gene corresponds to the Seq. Id. No. 2.

### Fermentation of the Recombinant Strains expressing the UPase enzyme or the PNPase enzyme

The recombinant strains called EXP05/03 (expressing the UPase enzyme) and EXP05/04 (expressing the PNPase enzyme) were separately fermented batchwise, using a fermenter with a useful volume of 15 liters, containing 15 1 of culture medium with the following composition (per liter):
3.2 g K₂HPO₄
0.6 g KH₂PO₄
20 g soytone;
36 g yeast extract;
1.0 g MgSO₄.7H₂O;
0.02 g kanamycin

The fermenter was inoculated with about 150 ml of bacterial suspension previously grown for about 24 h at 37°C. The fermentation was carried out using the following parameters: temperature 37°C, mechanical stirring at about 250 revolutions/minute, air flow automatically controlled to keep the pO₂ value at 20% of the saturation concentration, pH controlled at 7 ± 0.2 by addition of 10% ammonia solution or 20% phosphoric acid solution.

Once the fermentation (completed in about 24 hours) had been terminated the cell paste was collected by centrifugation, washed with 100 mM potassium phosphate buffer at pH 7.0, collected again by centrifugation and kept as wet cell paste at a temperature of -20°C.

### 4. Enzymatic activities of the Recombinant Strains expressing the UPase enzyme or the PNPase enzyme

The activities of cells obtained from 15 liters fermentations are reported

| STRAIN | UPase activity (U/g)* | PNPase activity (U/g)* |
|---|---|---|
| EXP05/03 F1 (UP) | 2000 | 6 |
| EXP05/03 F2 (UP) | 2000 | // |
| EXP05/04 F1 (PNP) | 10 | 400 |
| EXP05/04 F2 (PNP) | // | 245 |
| EXP05/04 F3 (PNP) | // | 340 |
| EXP05/04 F4 (PNP) | // | 340 |

| | | |
|---|---|---|
| *U/g = unit per gram of wet weight. | | |

The enzymatic activities of the recombinant strains are determined both singularly, by phosphorolysis reactions of natural substrates, and coupled with a standard reaction wherein a sugar is transferred from a pyrimidine base to a purine. The used reaction, which is basically a test of practical use, includes the transfer of the arabinose sugar from the Uracil pyrimidine base (Ara-U = sugar + base) to the Adenine purine base (with formation of Ara-A). This reaction is used because it is well known and described in the literature and functional from an operative point of view.

### 5. Determination of the enzymatic activity due to the UPase enzyme.

A known amount (100 or 200 microliters) of a suspension of UPase expressing cells, diluted 1:100 or 1:1000 as wet weight/volume in potassium phosphate buffer at pH 7.0-7.2, is added to 800 microliters of 75 mM Uridine solution in 100 mM phosphate buffer at pH 7.0-7.2, preincubated at 30°C. Exactly 5 minutes later, the phosphorolysis reaction is stopped by addition of 1 ml of 2N HCl. An aliquot of the reaction mixture is analyzed using a high performance liquid chromatograph (HPLC), equipped with a Nucleosil 100-5 (Macherey-Nagel) column, with a size of 250 x 4.6 mm. The elution is carried out using a 10 mM monobasic potassium phosphate - 6% methanol solution.

The enzymatic activity of the cell paste is expressed as unit/gram of wet weight (micromoles transformed each minute per 1 gram of wet cell paste) and is calculated with respect to a standard curve built with the amounts of uracil formed in the same conditions of the assay using increasing amounts of the same cell paste.

### 6. Determination of the enzymatic activity due to the PNPase enzyme.

A known amount (100 or 200 microliters) of a suspension of PNPase expressing cells , diluted 1:100 or 1:1000 as wet weight/volume in potassium phosphate buffer at pH 7.0-7.2, is added to 800 microliters of 60 mM Inosine solution in 100 mM phosphate buffer at pH 7.0-7.2, preincubated at 30°C. Exactly 10 minutes later, the phosphorolysis reaction is stopped by addition of 1 ml of 2N HCl. An aliquot of the reaction mixture is analyzed using a high performance liquid chromatograph (HPLC) equipped with a Nucleosil 100-5 column (Macherey-Nagel) with a size of 250 x 4.6 mm. The elution is carried out using 10 mM monobasic potassium phosphate - 6% methanol solution.

The enzymatic activity of the cell paste is expressed as unit/gram of wet weight (micromoles transformed each minute per 1 gram of wet cell paste) and is calculated with respect to a standard curve built with the amounts of ipoxantine formed in the same conditions of the assay using increasing amounts of the same cell paste.

### 7. Determination of the catalytic activity of the coupled UPase and PNPase enzymes.

The catalytic activity of the coupled UPase and PNPase enzymes in the mixture of cell suspensions before immobilization, in the washing waters after the immobilization and in the solid carrier is determined by a transglycosylation reaction carried out using standardized conditions.

50-100 microliters of the mixture of cell suspensions or of the washing waters after immobilization are added to 1 ml of reaction mixture while 200 or 400 mg (wet weight) of solid carrier having immobilized cells thereon containing the UPase enzymatic activity and the PNPase enzymatic activity are added to 10 ml of reaction mixture.

The reaction is carried out using the following solution: 40 mM arabinofuranosyl-uracil (Ara-U), 40 mM adenine, 30 mM monobasic potassium phosphate - pH 7.0-7.2 at a termostated temperature of 60°C. After 60 minutes at 60°C, the reaction is stopped by diluting the reaction in water 1:50 and cooling at 4°C. The percentage of adenine converted into arabinofuranosyl-adenine (ARA-A) is determined by analyzing an aliquot of the reaction mixtures with a high performance liquid chromatograph (HPLC) equipped with a Nucleosil 100-5 column (Macherey-Nagel) with a size of 250 x 4.6 mm, eluting with a 10 mM monobasic potassium phosphate - 6% methanol buffer. The catalytic activity of the coupled UPase and PNPase enzymes (transglycosylation catalytic activity) is expressed in unit/g (micromoles of Adenine converted each minute to form Ara-A in the conditions of the assay/gram of wet weight of cell paste) and is calculated with respect to the percentage of the adenine conversion.

### 8. Testing different resins for the enzyme immobilization

Immobilization tests with different contact times between the mixture of the cell suspensions and different types of resin used for the enzyme immobilization are performed.

The following resins were used (Rohm & Haas): Duolite A568; Duolite A7; Amberlite FPA54; Amberlite FPC3500; Amberlite XAD761, Dowex MWA1, Amberlyst 21, Diaion WA30, Amberlite IRA67.

10 ml of a mixture of cell suspensions in 0.5 M potassium phosphate buffer at pH 7.5 with UPase enzymatic activity concentration of about 67 unit/ml and PNPase enzymatic activity of about 23 unit/ml were added to 1 dry gram of each resin (2 wet grams for Amberlite IRA67, supplied in this form). The catalytic activity of the two coupled phosphorylases was about 2.65 U/ml.

The incubation mixtures were left upon gentle stirring for about 72 hours.

Resins were filtered and analyzed using standard reactions (paragraph 7)

| RESIN | Carrier matrix | Functional group | Capacity | Recovered amounts | specific activity | % uptake |
|---|---|---|---|---|---|---|
| Duolite A7 | | | | 1.3 g | 3.1 U/g | 15.7% |
| Duolite A568 | Phenol-formaldheyde | Tertiary amine | 1.2 meq/ ml 3.9 meq/g | 2.6 g | 6.6 U/g | 64.7% |
| Amberlite FPA54 | | | | 1.3 g | 2.2 U/g | 11.5% |
| Amberlite FPC3500 | | | | 2.5 g | 0.2 U/g | 2.3% |
| Amberlite XAD761 | | | | 1.3 g | 0.3 U/g | 1.6% |
| Dowex MWA1 | Styrene-divinylbenzene | Tertiary amine | 1.0 meq/ ml 3.5 meq/g | 1.3 g | 2.8 U/g | 13.7% |
| Amberlyst 21 | Styrene-divinylbenzene | Alkylam ine | 1.5 meq/ ml 4.7 meq/g | 1.3 g | 2.4 U/g | 11.8% |
| Diaion WA30 | Styrene-divinylbenzene | Tertiary amine | 1.5 meq/ ml 3.0 meq/g | 1.3 g | 3.0 U/g | 14.7% |
| Amberlite IRA67 | Acrylate-divinylbenzene | Tertiary amine | 1.6 meq/ ml 5.6 meq/g | 1.3 g | 0.6 U/g | 2.9% |

Duolite A568 proved to be the best resin with a percentage of activity immobilization on the resin higher then 60% in the described conditions.

### 9. Co-immobilization of Recombinant Strains expressing the UPase enzyme and the PNPase enzyme

The better conditions for the co-immobilization of genetically modified cells on a solid carrier were then studied.

### 9.1 Ratio between the UPasic and PNPasic enzymatic activities

The availability of cells containing high amounts of UPase and PNPase allows the modulation of the two enzymatic activities to optimize the reaction catalyzed by the coupling of the two enzymes.

Cell suspension mixtures of the two strains were prepared in such a way to have activity ratios (expressed in unit/wet gram) according to paragraphs 5. and 6. equal to:
UP/PNP=0.5; UdP/PNP=1; UdP/PNP=2; UdP/PNP=3; UdP/ PNP=6

The suspension mixtures were tested for the ability to perform a standard reaction with all amounts being equal (see paragraphs 6. and 7.)

The best condition proved to be that with a UdP/PNP ratio equal to 3, slightly higher with respect to the UdP/PNP ratio equal to 2, which is however preferred since it needs a lower amount of cell paste with PNPase (the PNPase enzyme is expressed in a lower amount with respect to the UPase enzyme).

### 9.2 Concentration of the UPase enzymatic activity

Immobilization tests with different concentrations of UPase in the mixture of cell suspensions comprised between 30 and 150 U/ml of suspension (in the conditions of the test described in example 5) are carried out. The best condition regarding the activity of the immobilized carrier and the activity lost in the washing is that with about 75 U/ml of suspension.

### 9.3 Concentration of the PNPase enzymatic activity

Immobilization tests with different concentrations of PNPase in the mixture of cell suspensions comprised between 15 and 75 U/ml of suspension (in the conditions of the test described in the example 6) are carried out. The best condition regarding the activity of the immobilized carrier and the activity lost in the washing is that with about 25 U/ml of suspension.

### 9.4 Ratio between UPase and PNPase enzymatic activity and resin amount

Immobilization tests with ratios between UPase and PNPase enzymatic activity of the mixture of cell suspensions and resin amount comprised between 330 and 1330 Unit/dry gram of resin for UPase and between 150 and 660 Unit/dry gram of resin for PNPase are carried out.

The best condition regarding the activity of the immobilized carrier and the activity lost during the washing is given by the immobilization of a mixture of cell suspensions with about 750 Unit/dry gram of resin for UPase and about 250 Unit/dry gram of resin for PNPase.

### 9.5. Concentration of the catalytic activity of the two coupled phosphorylases

Immobilization tests with concentrations of catalytic activity of the two coupled phosphorylases (in the conditions of the test described in the example 7) comprised between 1 unit/ml cell suspension mixture and 8 unit/ml cell suspension mixture are performed. The best condition for the activity of the immobilized resin is that with 5 unit/ml of cell suspension mixture.

### 9.6. Ratio between the catalytic activity of the two coupled phosphorylases and the amount of dry resin

Immobilization tests with ratios between the catalytic activity of the two coupled phosphorylases and the resin amount comprised between 30 and 80 unit/gram of dry resin are performed (selected condition: about 50 unit/dry gram of resin).

The best condition regarding the activity of the immobilized resin and the activity lost in the washing is that with 50 units per dry gram of resin.

### 9.7. Ratio between cell amount and resin weight

Immobilization tests with ratios between cell amount (as wet weight) and resin amount (as dry weight) comprised between 0.5:1 and 2:1 are performed.

The best condition was found to be that with a 1:1 ratio between cell paste weight and resin weight.

### 9.8. Salt concentration of the immobilization buffer

Immobilization tests with cell suspension mixtures in buffers with different monobasic potassium phosphate molarity at pH 7.4, the physiological and optimum pH for the enzymatic activities, are performed. The suspensions were prepared in 0 M; 0.1 M; 0.5 M and 1 M buffers.

The selected condition is the one which involves the use of 0.5 M monobasic potassium phosphate buffer.

### 9.9. Immobilization Time

Immobilization tests with different contact times between the mixture of cell suspensions and the resin are performed. Periods of 24 hours, 48 hours, 72 hours, 6 days and 2 weeks were used. The best results are obtained leaving the cell suspension mixture in incubation with the resin for not less than 72 hours. Neither decays of the enzymatic activity nor other contraindications following so long immobilization times as up to two weeks are noted.

The resin is then analyzed regarding to the main application parameters.

### 10. pH range

Tests are performed, which involve carrying out subsequent reactions at different pH values to evaluate the range in which the enzymatic activity of the resin is reusable.

Standard reactions were carried out (see paragraph 7) at pH values equal to:
4.0; 5.0; 6.0; 7.0; 8.0; 9.0; 10.0

Additional subsequent reactions for each pH value reported above were carried out to evaluate, in each case, the maintenance of the activity with respect to the first reaction. The enzymatic activity of the immobilized cells is maintained in the pH range comprised between pH 5.0 and pH 9.0 and preferably between pH 6.0 and pH 9.0.

### 11. Inactivation temperature

Tests are performed, which involve carrying out subsequent reactions at different temperature values to verify the biocatalyst inactivation. Standard reactions at 70°C, 75°C and 80°C temperature were carried out (see paragraph 7). The enzymatic activity is maintained in subsequent reactions also at 70°C while it decreases at 75°C (the 50% of the activity is lost) and it is completely lost at 80°C.

### 12. Co-immobilization of cells containing the UPase enzyme and cells containing the PNPase enzyme

The immobilization of cell suspensions containing the UPase enzymatic activity and the PNPase enzymatic activity is prepared starting from a mixture of cell suspensions prepared in such a way to have a ratio between the enzymatic activity of the UPase enzyme and the enzymatic activity of the PNPase enzyme comprised between 1:1 and 3: 1. In this example the co-immobilization of a cell suspension mixture is described wherein the ratio between the enzymatic activity of the UPase enzyme and that of the PNPase enzyme is about 3:1.

About 19 grams of Duolite A568 Rohm & Haas resin (dry weight) are added to 190 ml of a mixture of cell suspensions composed of 6.25 grams of EXP05/03 (UP) cells as wet weight and 18.2 grams of EXP05/04 (PNP) cells.

The cell suspension had been prepared in 0.5 M potassium phosphate buffer at pH 7.5 and had a UPase enzymatic activity concentration equal to about 115 unit/ml and a PNPase enzymatic activity equal to about 33 unit/ml. The catalytic activity of the two coupled phosphorylases was about 4.7 U/ml.

The cells/resin suspension is kept at room temperature under gentle stirring for 48 hours. The immobilized resin is then vacuum filtered and thoroughly washed with deionized water until clear washing waters are obtained (about 2 liters).

About 65 grams of wet resin, with a specific activity of the coupled phosphorylases equal to about 7.5 U/wet gram and with an immobilization of more than 50% of the initial activity, were recovered.

The resin with the immobilized enzymatic activities is then kept at 4°C in 0.1 M potassium phosphate buffer at pH 7.5.

### 13. Bioconversion reactions catalyzed by resin having UPase and PNPase containing cells co-immobilized thereon

### a) Preparation of Adenosine

10 ml of a solution of 60 mM 2'-deoxyUridine and 20 mM adenine in 30 mM potassium phosphate buffer at pH 7.1 are added to 0.2 grams of resin (wet weight) with a catalytic activity of the coupled UPase and PNPase enzymes equal to about 7.5 unit/gram, calculated as in paragraph 7, the resin having just been filtered and washed with deionized water. : After 1 day at 60°C, the resin with the immobilized biocatalyst is separated and the filtered material is processed for purifying the Adenosine.

The resin can be immediately re-used for a subsequent reaction or can be recovered and kept at 4°C in 0.1 M potassium phosphate buffer until the successive use.

The bioconversion yield was about 80%.

### b) Preparation of arabinofuranosyl-DiaminoPurine (Ara-DAMP)

10 ml of a solution with the following composition: 60 mM Ara-U (beta-D-arabinofuranosyl-uracil); 20 mM DiaminoPurine; in 30 mM potassium phosphate buffer at pH 7.1, are added to 0.2 grams of resin (wet weight) with a catalytic activity of the coupled UPase and PNPase enzymes equal to about 7.5 unit/gram (paragraph 7), the resin having just been filtered and washed with deionized water.,

After 1 day at 60°C, the resin with the immobilized biocatalyst is separated and the filtered material is processed for the Ara-DAMP purification.

The resin with the immobilized biocatalyst can be immediately re-used for a subsequent reaction or can be recovered and kept at 4°C in 0.1 M potassium phosphate buffer until the successive use. The bioconversion yield was about 80%.

### c) Preparation of 2'-Deoxyadenosine

10 ml of a solution with the following composition: 60 mM 2'-deoxyUridine; 20 mM Adenine; in 30 mM potassium phosphate buffer at pH 7.1 are added to 0.2 grams of resin (wet weight) with a catalytic activity of the coupled UPase and PNPase enzymes equal to about 7.5 unit/gram (paragraph 7), the resin having just been filtered and washed with deionized water.

After 1 day at 60°C, the resin with the immobilized biocatalyst is separated and the filtered material is processed for the purification of 2'-Deoxyadenosine.

The resin with the immobilized biocatalyst can be immediately re-used for a subsequent reaction or can be recovered and kept at 4°C in 0.1 M potassium phosphate buffer until the successive use. The bioconversion yield was about 80%.

### d) Preparation of 2'-deoxyGuanosine

10 ml of a solution with the following composition: 20 mM 2'-deoxyUridine; 5 mM Guanine; in 30 mM potassium phosphate buffer at pH 7.1 are added to 0.2 grams of resin (wet weight) with a catalytic activity of the coupled UPase and PNPase enzymes equal to about 7.5 unit/gram (paragraph 7), the resin having just been filtered and washed with deionized water.,

After 1 day at 60°C, the resin with the immobilized biocatalyst is separated and the filtered material is processed for the purification and precipitation of 2'-deoxyGuanine.

The resin with the immobilized biocatalyst can be immediately re-used for a subsequent reaction or can be recovered and kept at 4°C in 0.1 M potassium phosphate buffer until the successive use. The bioconversion yield was about 80%.

### e) Preparation of 2'-deoxyDiaminopurine

10 ml of a solution with the following composition: 20 mM 2'-deoxyUridine; 5 mM 2'-deoxyDiaminopurine; in 30 mM potassium phosphate buffer at pH 7.1 are added to 0.2 grams of resin (wet weight) with a catalytic activity of the coupled UPase and PNPase enzymes equal to about 7.5 unit/gram (paragraph 7), the resin having just been filtered and washed with deionized water.,

After 1 day at 60°C, the resin with the immobilized biocatalyst is separated and the filtered material is processed for the purification of 2'-deoxyDiaminopurine.

The resin with the immobilized biocatalyst can be immediately re-used for a subsequent reaction or can be recovered and kept at 4°C in 0.1 M potassium phosphate buffer until the successive use. The bioconversion yield was about 80%.

### f) Preparation of Mizoribine (Bredinine)

10 ml of a solution with the following composition: 60 mM Uridine; 20 mM carbamoyl-Imidazolone; 30 mM potassium phosphate buffer at pH 7.1 are added to 0.2 grams of resin (wet weight) with a catalytic activity of the coupled UPase and PNPase enzymes equal to about 7.5 unit/ gram (paragraph 7), the resin having just been filtered and washed with deionized water.,

After 1 day at 60°C, the resin with the immobilized biocatalyst is separated and the filtered material is processed for the purification of Mizoribine.

The resin with the immobilized biocatalyst can be immediately re-used for a subsequent reaction or can be recovered and kept at 4°C in 0.1 M potassium phosphate buffer until the successive use. The bioconversion yield was about 80%.

### g) Preparation of arabinofuranosyl-Adenine (Ara-A)

10 ml of a solution with the following composition: 40 mM Ara-U; 40 mM Adenine; in 30 mM potassium phosphate buffer at pH 7.1 are added to 0.2 grams of resin (wet weight) with a catalytic activity of the coupled UPase and PNPase enzymes equal to about 7.5 unit/gram (paragraph 7), the resin having just been filtered and washed with deionized water. ,

After 1 day at 60°C, the resin with the immobilized biocatalyst is separated and the filtered material is processed for the purification of Ara-A.

The resin with the immobilized biocatalyst can be immediately re-used for a subsequent reaction or can be recovered and kept at 4°C in 0.1 M potassium phosphate buffer until the successive use. The bioconversion yield was about 70%.

### 14. Reactions to Fludarabine with cell recombinant resin containing co-immobilized UPase and PNPase

500 ml of a solution at 60°C with the following composition: 75 mM Ara-U; 50 mM Adenine; in 30 mM potassium phosphate buffer at pH 7.1 containing 30% DMSO are added to 18 grams of resin (wet weight) with a catalytic activity of the coupled UPase and PNPase enzymes equal to about 12.7 unit/gram (paragraph 7), the resin having just been filtered and washed with deionized water.,

The reaction is performed at 60°C for 20 h, then the resin with the immobilized biocatalyst is separated and the filtered material is processed for the purification of Fludarabine.

The resin with the immobilized biocatalyst is immediately re-used for a subsequent reaction performed with the same methods. 10 consecutive reactions are performed re-using the same resin.

| **No. subsequent reaction (20 h)** | **2-F-Adenine →Fludarabine** | **Ara-U → Uracil** |
|---|---|---|
| 1° Reaction | 82.1% | 54.0% |
| 2° Reaction | 81.4% | 53.5% |
| 3° Reaction | 82.5% | 54.4% |
| 4° Reaction | 82.0% | 54.0% |
| 5° Reaction | 78.0% | 54.5% |
| 6° Reaction | 83.7% | 54.8% |
| 7° Reaction | 80.5% | 55.6% |
| 8° Reaction | 82.1% | 54.3% |
| 9° Reaction | 80.4% | 51.5% |
| 10° Reaction | 80.0% | 52.1% |

The resin in these conditions is able to perform at least 10 reactions at a high temperature with high concentrations of solvent with a 10 days long permanence at 60°C. The activity loss is negligible small or in any case quite limited. Thus a biocatalyst of notable industrial interest for the production of nucleosides and analogous thereof and generally for bioconversion reactions is available.

### Bibliographic references:

1. Ramakrishna S.V., Prakasham R.S. "Microbial fermentations with immobilized cells", ...Current science., 1999, 77 Vol 1, 87-100.
2. Novarro J.M., Durand G., Eur. J. Appl. Microbiol., 1977, 4, 243-254.
3. Kennedy J.F., Cabral J.M.S., Immobilized Cells and Enzymes (ed. Woodward, J.) IRL Press, 1985, 19-37.
4. Chibata I et al, JP 7425189, 1974.
5. Hottori T., Furusaka C., J. Biochem., 1960, 48, 821-837.
6. Hottori T., Furusaka C., J. Biochem., 1961, 50, 312-315.
7. Porter J.T., Short S.A., Journal Biological Chemistry, 1995, 270 (26), 15557-15562, 1995.
8. Okuyama K., Noguchi T., Bioscience Biotechnology and Biochemistry, 2000, 64 (10) 2243-2245.
9. Hutchinson D.W., Trends Biotechnolog., 1990, 8, 348-353.
10. Hanrahan J.R., Hutchinson D.W., J. Biotechnol., 1992, 23, 193-210.
11. Krenitsky T.A., 1979, EP 0 002 192.
12. Krenitsky T.A. et al., Biochemistry, 1981, 20, 3615-3621.
13. Krenitsky T.A. et al., Carbohydrate Research, 1981, 97, 139-146.
14. Krenitsky T.A. et al., J. Med. Chem., 1986, 29,138-143.
15. Takehara M. et al., Bioscience Biotechnology and Biochemistry, 1995, 50(10), 1987-1990.
16. Bestetti G. et al., US 6,911,341 B2.
17. Utagawa T. et al., Agric. Biol. Chem., 1985, 49, 3239-3246.
18. Utagawa T. et al., Molec. Catalysis Enzymatic., 1999, 6 (3-4), 215-222.
19. Cotticeli G. et al., Nucleosides & Nucleotides, 1999, 18 (4-5), 1135-1136.

## Claims

1. A process for the production of nucleosides, comprising the reaction of a pentose-1-phosphate with a purine or pyrimidine base, **characterized in that** said reaction is catalyzed by Uridine Phosphorylase (UPase) and/or Purine Nucleoside Phosphorylase (PNPase) producing cells, which have been immobilized by adsorbing said cells on a weak anionic exchange resin, wherein said cells are Escherichia coli cells and said resin is Duolite A568®.

2. A process for nucleosides transglycosylation, comprising the reaction between
a) a pyrimidine nucleoside and a purine base or
b) between a purine nucleoside and a pyrimidine base or
c) between a purine base and a purine nucleoside containing a different purine base or
d) between a pyrimidine base and a pyrimidine nucleoside containing a different pyrimidine base,
wherein said reaction is catalyzed by UPase producing cells and by PNPase producing cells or by UPase and PNPase producing cells, **characterized in that** said cells are immobilized by adsorbing said cells on a weak anionic exchange resin, wherein said immobilized cells are UPase producing Escherichia coli cells and PNPase producing Escherichia coli cells and said resin is Duolite A568®.

3. A process according to claim 2, wherein said reaction occurs between 2'-fluoroadenine and arabinofuranosyl-uracil and leads to Fludarabine.

4. A process according to claim 1 or 2, wherein said Escherichia coli cells are cells of DH5alpha strain transformed with the plasmid vectors having the sequences reported in Seq. Id. No. 1 and 2.

5. A process according to claim 4, wherein said cells are co-immobilized on the resin.

6. A process according to any one of the preceding claims, wherein the ratio between the wet weight of said cells and the dry weight of said resin is comprised between 0.5:1 and 2:1 and is preferably about 1:1.

7. A process according to any one of the preceding claims, **characterized in that** two types of different cells are immobilized at the same time, wherein said two types of cells are UPase producing Escherichia coli cells and PNPase producing Escherichia coli cells.

## Patentansprüche

1. Verfahren zur Herstellung von Nukleosiden, umfassend die Reaktion eines Pentose-1-phosphats mit einer Purin- oder Pyrimidinbase, **dadurch gekennzeichnet, dass** die Reaktion durch Uridinphosphorylase (UPase)- und /oder Purinnucleosidphosphorylase (PNPase)-herstellende Zellen katalysiert wird, die durch Adsorbieren der Zellen auf einem schwachen Anionenaustauschharz immobilisiert worden sind, wobei die Zellen Escherichia coli-Zellen sind und das Harz Duolite A568^{®} ist.

2. Verfahren zur Nucleosidtransglycosylierung, umfassend die Reaktion zwischen
a) einem Pyrimidinnucleosid und einer Purinbase oder
b) zwischen einem Purinnucleosid und einer Pyrimidinbase oder
c) zwischen einer Purinbase und einem eine unterschiedliche Purinbase enthaltenden Purinnucleosid oder
d) zwischen einer Pyrimidinbase und einem eine unterschiedliche Pyrimidinbase enthaltenden Pyrimidinnucleosid,
wobei die Reaktion durch UPase-herstellende Zellen und durch PNPase-herstellende Zellen oder durch UPase- und PNPase-herstellende Zellen katalysiert wird, **dadurch gekennzeichnet, dass** die Zellen durch Adsorbieren der Zellen auf einem schwachen Anionenaustauschharz immobilisiert sind, wobei die immobilisierten Zellen UPase-herstellende Escherichia coli-Zellen und PNPase-herstellende Escherichia coli-Zellen sind und das Harz Duolite A568^{®} ist.

3. Verfahren gemäß Anspruch 2, wobei die Reaktion zwischen 2'-Fluoradenin und Arabinfuranosyluracil stattfindet und zu Fludarabin führt.

4. Verfahren gemäß Anspruch 1 oder 2, wobei die Escherichia coli-Zellen Zellen des Stamms DH5alpha sind, der mit den Plasmidvektoren transformiert ist, die die in Seq. Id. No. 1 und 2 wiedergegebenen Sequenzen enthalten.

5. Verfahren gemäß Anspruch 4, wobei die Zellen auf dem Harz coimmoblisiert sind.

6. Verfahren gemäß einem der vorangegangenen Ansprüche, wobei das Verhältnis zwischen dem Nassgewicht der Zellen und dem Trockengewicht des Harzes zwischen 0,5:1 und 2:1 liegt und bevorzugt ungefähr 1:1 ist.

7. Verfahren gemäß einem der vorangegangenen Ansprüche, **dadurch gekennzeichnet, dass** zwei Typen unterschiedlicher Zellen zur selben Zeit immobilisiert sind, wobei die zwei Zelltypen UPase-herstellende Escherichia coli-Zellen und PNPase-herstellende Escherichia coli-Zellen sind.

## Revendications

1. Procédé pour la production de nucléosides, comprenant la réaction d'un pentose-1-phosphate avec une base purique ou pyrimidique, **caractérisé en ce que** ladite réaction est catalysée par des cellules productrices d'uridine phosphorylase (UPase) et/ou de purine nucléoside phosphorylase (PNPase), qui ont été immobilisées par adsorption des dites cellules sur une résine anionique faible échangeuse, dans lequel lesdites cellules sont des cellules d'Escherichia coli, et ladite résine est la Duolite A568®.

2. Procédé pour la transglycosilation de nucléosides, comprenant la réaction entre :
a) un nucléoside pyrimidique et une base purique, ou
b) entre un nucléoside purique et une base pyrimidique, ou
c) entre une base purique et un nucléoside purique contenant une base purique différente, ou
d) entre une base pyrimidique et un nucléoside pyrimidique contenant une base pyrimidique différente,
dans lequel ladite réaction est catalysée par des cellules productrices d'UPase et des cellules productrices de PNPase, ou par des cellules productrices d'UPase et de PNPase, **caractérisé en ce que** lesdites cellules sont immobilisées par adsorption des dites cellules sur une résine anionique faible échangeuse, dans lequel lesdites cellules immobilisées sont des cellules d'Escherichia coli productrices d'UPase et des cellules d'Escherichia coli productrices de PNPase, et ladite résine est la Duolite A568®.

3. Procédé selon la revendication 2, dans lequel ladite réaction se produit entre la 2'-fluoroadénine et l'arabinofurannosyl-uracile, et aboutit à la fludarabine.

4. Procédé selon la revendication 1 ou 2, dans lequel lesdites cellules d'Escherichia coli sont des cellules de la souche DH5 alpha, transformées avec les vecteurs plasmidiques ayant les séquences mentionnées dans les Seq. Id. N° 1 et 2.

5. Procédé selon la revendication 4, dans lequel lesdites cellules sont immobilisées ensemble sur la résine.

6. Procédé selon l'une quelconque des revendications précédentes, dans lequel le rapport entre le poids à l'état humide des dites cellules et le poids à sec de ladite résine, est compris entre 0,5 : 1 et 2 : 1, et est, de préférence, d'environ 1:1.

7. Procédé selon l'une quelconque des revendications précédentes, **caractérisé en ce que** deux types de cellules différentes sont immobilisés en même temps, les deux types de cellules étant des cellules d'Escherichia coli productrices d'UPase et des cellules d'Escherichia coli productrices de PNPase.
